# EUROPEAN PATENT SPECIFICATION

(11) **EP 1 737 955 B1**
(45) Date of publication and mention of the grant of the patent: **31.12.2008**
(21) Application number: 04725108.7
(22) Date of filing: 01.04.2004
(51) Int. Cl.: C12N 15/11

(54) **NOVEL TEMPERATURE REGULATED PROMOTERS AND EXPRESSION VECTORS FROM S.POMBE**
NEUE TEMPERATUR-REGULIERTE PROMOTOREN UND EXPRESSIONSVEKTOREN AUS S.POMBE
PROMOTEURS REGULES DE TEMPERATURE ET VECTEURS D'EXPRESSION DE S.POMBE

(43) Date of publication of application: 03.01.2007
(73) Proprietor: Council of Scientific and Industrial Research, New Delhi 100 001 (IN)
(72) Inventor: SINGH, Jagmohan, 160 036 Chandigarh (IN); KUMAR, Raj, 160 036 Chandigarh (IN)
(74) Representative: Lee, Nicholas John
(86) International application number: PCT/IB2004/000989
(87) International publication number: WO 2005/095608

(56) References cited:
- BELLEMARE D R ET AL: "A novel copper-regulated promoter system for expression of heterologous proteins in Schizosaccharomyces pombe" GENE: AN INTERNATIONAL JOURNAL ON GENES AND GENOMES, ELSEVIER SCIENCE PUBLISHERS, BARKING, GB, vol. 273, no. 2, 8 August 2001 (2001-08-08), pages 191-198, XP004302702 ISSN: 0378-1119
- IACOVONI J S ET AL: "A new inducible protein expression system in fission yeast based on the glucose-repressed inv1 promoter" GENE, ELSEVIER BIOMEDICAL PRESS. AMSTERDAM, NL, vol. 232, no. 1, 17 May 1999 (1999-05-17), pages 53-58, XP004166802 ISSN: 0378-1119
- SIAM R ET AL: "Choosing and using Schizosaccharomyces pombe plasmids" METHODS : A COMPANION TO METHODS IN ENZYMOLOGY, ACADEMIC PRESS INC., NEW YORK, NY, US, vol. 33, no. 3, July 2004 (2004-07), pages 189-198, XP004509884 ISSN: 1046-2023
- MAUNDRELL K: "nmt1 of fission yeast. A highly transcribed gene completely repressed by thiamine" JOURNAL OF BIOLOGICAL CHEMISTRY, AMERICAN SOCIETY OF BIOLOGICAL CHEMISTS, BALTIMORE, MD, US, vol. 265, no. 19, 5 July 1990 (1990-07-05), pages 10857-10864, XP002211007 ISSN: 0021-9258

## Description

### FIELD OF INVENTION

The present invention relates to novel temperature promoters and set of expression vectors isolated from *Schizosaccharomyces pombe.* The vectors so developed can be used for regulated expression of proteins, both homologous and heterologous, very efficiently and economically.

### BACKGROUND INFORMATION

Early search for new promoters in *S. pombe* yielded *adh*1, a strong but constitutive promoter, which drives the expression of the glycolytic enzyme alcohol dehydrogenase. This promoter has been used in the vectors pART1 and pEVP11 (Russell, 1989, In: Molecular Biology of the Fission Yeast. San Diego: Academic Press, Inc. Nasim. A., Young, P., and Johnson, B.F., Eds. pp. 243-71). Among the inducible promoters are the glucose regulated promoter fructose bisphosphase (*fbp*) (Hoffman and Winston, 1989, Gene 84: 473-79) and the invertase promoter (*inv*1) (Tanaka et al., 1998, Biochem. Biophys. Res. Commun. 245: 246-53), which have been used successfully to express a number of proteins including GFP. The strongest known promoter in *S. pombe* is *adh*1, which is a constitutively expressed promoter. Although expression driven by this promoter can yield levels of protein to the extent of 0.5-2% of total cellular protein (Russell, 1989, In Molecular Biology of the Fission Yeast. San Diego: Academic Press, Inc. Nasim. A., Young, P., and Johnson, B.F., Eds. pp. 243-271), it can pose difficulties when the expression of toxic proteins is desired. Therefore, researchers have always focused their attention to the development of inducible promoters.

The regulatable promoters in *S. pombe* include the *fbp, inv*1 and *nmt*1. The *fbp*1 promoter is repressed by 8% glucose and derepressed in 0.1% glucose plus 3% maltose (Hoffman and Winston, 1989, Gene 84: 473-479). A limitation of this promoter is that it is derepressed in stationary phase and furthermore, the cells expressing the vector do not grow well under the inducing conditions. A similar drawback is faced by the *inv*1 promoter derived from the *inv*1 gene that codes for invertase in *S. pombe* (Tanaka et al., 1998, Biochem. Biophys. Res. Commun. 245: 246-53). It is also repressed by glucose and derepressed by depletion of glucose. The regulation of expression using this promoter cannot be very tight because of progressive depletion of glucose in the culture medium as a result of its utilization during the cellular growth. Likewise another inducible promoter of *S. pombe* acid phosphatase structural gene (*pho*1), which is induced by low concentrations of inorganic phosphate in the medium, has the drawback that it shows a significant level of uninduced transcription, thus negating its potential use as a promoter (Maundrell, 1990, J. Biol. Chem. 265: 10857-64). Thus, till date only one promoter element has come to be used regularly as a research tool, namely *nmt*1, which is repressed by high concentration of thiamin and induced by absence of thiamin (Maundrell, 1990, J. Biol. Chem. 265: 10857-64). The most common and the strongest form of this promoter is *nmt*1. A few derivatives of the *nmt*1 promoter were subsequently developed that yield very high (*nmt*1), medium (*nmt*41) and low (*nmt*81) levels of expression (Forsburg, 1993, Nucleic Acid Res. 21: 2955-56). A related problem of these promoters is their leakiness even under repressed conditions and the leakiness appears to be directly proportional to the promoter strength (Forsburg, 1993, Nucleic Acid Res. 21: 2955-2956). The induction regime involves growth of cells harboring the plasmid expressing a particular gene under the control of the *nmt*1 promoter in presence of thiamin. After growing to early log phase (OD₆₀₀ of ∼0.3), cells are washed with and transferred to a synthetic medium lacking thiamin and grown further. The expression of the gene of interest is observed after nearly 18-20 hours of growth in the medium lacking thiamin. Apart from the cumbersome problem of handling cells under sterile conditions through the steps of washing and resuspension in thiamin-free medium, the other major problem with this promoter is that it is leaky, that is, the expression of the gene is never completely repressed in the presence of thiamin. This can lead to a deleterious effect on the growth rate of cells even before the start of induction because of possible metabolic load. A similar effect may be exerted during induction because of the long time of induction to achieve full expression level. The presence of the heterologous protein during the long induction period in the intracellular milieu may also lead to cellular defect and protein degradation. The present invention therefore obviates these drawbacks and through the process of this invention a temperature sensitive (or regulated) promoter based vector for expression of heterologous proteins in fission yeast, *Schizosaccharomyces pombe* has been developed. This invention is particularly useful in efficient, economic and regulated expression of proteins both homologous and heterologous.

The new promoter elements are isolated by screening of promoters which allow expression of a Green fluorescent protein (GFP) reporter gene in response to a shift in temperature. The promoter elements thus isolated represent a truncated region of the previously reported no-message for thiamine 1 (*nmt*1) promoter (Maundrell, 1990, J. Biol. Chem. 265: 10857-64) having some unique properties that make them more advantageous to use as compared to other known promoters including *nmt*1 in *Schizosaccharomyces pombe (S. pombe)*. These characteristics are: i) temperature sensitive expression: induction of expression by shifting the temperature from 36°C to 25°C, ii) faster kinetics of expression, iii) moderate level of expression, iv) low leaky expression and v) lack of toxicity.

Selection of a suitable promoter is the most important factor in obtaining optimum level of expression. In early studies, several promoters that work in *Saccharromyces cerevisiae* (*S. cerevisiae*) were tried in S. *pombe,* but with limited success. The promoters that provided a good level of expression included phosphoglycerate kinase (*PGK*), alcohol dehydrogenase I (*ADH*1) and iso-1-cytochrome c (*CYC)*. Among other heterologous promoters that work in *S. pombe* are the simian virus (SV40) promoter, human cytomegalovirus (hCMV) promoter, cauliflower mosaic virus (CaMV) 35S promoter, tomato nitrate reductase promoter, human serum albumin TATA element, adenovirus region 3 promoter, human immunodeficiency virus-1 Long terminal repeat (HIV-1 LTR) promoter etc. Another expression system combines the CaMV promoter and tetracycline regulatory sequences to elicit tetracycline regulated expression. Recently, a co-transformation strategy using a vector containing the hCMV promoter and another vector containing the autonomous replication sequence (*ars*1) and stable (stb) elements has been reported {review by Giga-Hama, 1997, in Foreign Gene Expression in Fission Yeast Schizosaccharomyces pombe (Giga-Hamma and Kumagai, Eds.), Springer-Verlag, Berlin pp. 3-28}.

The advantages of the new promoters vis-a-vis the nmt1 and its other known derivative promoters strongly support the aspects of novelty and lack of obviousness and anticipation. In fact, literature published on the characteristics of the mnt1 promoters have only focussed on the minimal size of the nmt1 promoters that is repressible by thiamin and on the role of trans-acting factors. No investigation has envisaged or anticipated in published literature whether derivatives of the nmt1 promoter having altogether new characteristics different from the original parent promoter could be derived. In fact, from our screen we were expecting to isolate heat-inducible promoters, like heat shock promoters, rather than the temperature sensitive ones that we actually obtained. Thus, identification of promoter that is heat sensitive, was an unexpected discovery for us as well. Therefore, we believe, that these points will strengthen the claims for lack of obviousness and anticipation in obtaining the present set of promoters.

### OBJECTS OF THE INVENTION

The main objective of the present invention, therefore, is to develop expression vectors based on novel promoter elements isolated from *Schizosaccharomyces pombe.*

Another objective of the invention is to develop vectors with regulatable promoters, which are regulated by temperature shift.

Yet another objective of the invention is to use these vectors as substitutes for the *nmt*1 promoter based vector.

Yet another objective of this invention is to develop promoters with faster kinetics of induction of expression.

Yet another objective of this invention is to develop alternative, simpler, cheaper and user-friendly modes of induction that are easier to perform as compared to *nmt*1.

Yet another objective of this invention is to develop expression systems in which heterologous protein expression does not affect the viability of the host cells. Therefore the present invention relates to expression vectors based on novel regulatable promoter elements isolated from *Schizosaccharomyces pombe* which comprise:
(i) Construction of a fission yeast promoter library wherein a partial *Sau*3AI library of genomic DNA was cloned upstream of the GFP reporter gene, using known procedures,
(ii) Screening of *S. pombe* promoter library using known procedure wherein cells plated on to suitable plates were examined under reflected UV light to monitor expression of the GFP reporter gene under different conditions of growth,
(iii) Isolating from the genomic library as constructed in step (i), two clones having genomic DNA fragment of *S. pombe,* allowing GFP expression,
(iv) using the said clónes to regulate the GFP expression by temperature shift,
(v) Sequencing the genomic DNA fragments as obtained in the step (iii) above as novel promoter elements which were 185 and 146 bases long,
(vi) Construction of new vectors pJRK2 and pJRK3 by cloning the promoter elements as obtained in step (v) above, respectively. These two vectors have been deposited in International Depository Authority on Microbial Type Culture Collection and have been given accession nos. MTCC 5106 and MTCC 5107, respectively,
(vii) Characterization of the promoter elements as *nmt-185* and *nmt-146,*
(viii) Determining the strength of new promoters using GFP and β-galactosidase as reporter genes, and
(xi) Using the promoters to express other genes like streptokinase and *cdc18.*

### BRIEF DESCRIPTION OF THE ACCOMPANYING DRAWINGS/FIGURES

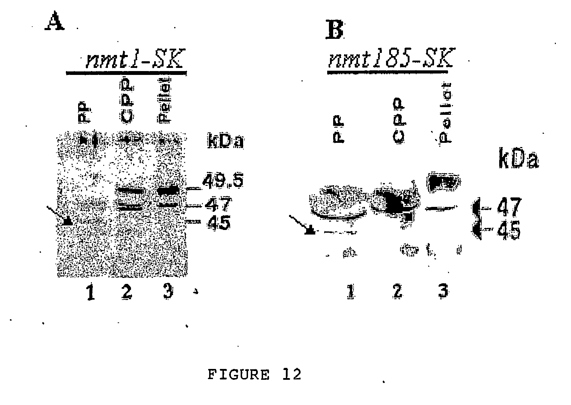

**Fig. 1** DNA sequences of *nmt 1, nmt-185* and *nmt-146* promoter. In the sequence of *nmt1* promoter, the horizontal arrow indicates the site of transcription inititation and ththis site occurs 27 base pairs downstream of the sequence TATATAAA in the red background.
**Fig.2** New vector having Accession No. MTCC 5106 harbouring *nmt-185* promoter.
**Fig.3** New vector having Accession No. MTCC 5106 harbouring *nmt-146* promoter
**Fig.4** **GFP expression under the control of *nmt1* and *nmt-185* promoters**
   Wild type *S*. *pombe* strains expressing GFP gene under the control of the *nmt1* or *nmt-185* promoters were streaked on PMA-leu plate in presence and absence of thiamine at 37°C or 25°C. After 2 days of incubation, photograph was taken under reflected UV light.
**Figure 5** **GFP expression under the control of *nmt1* promoter was monitored at cellular level by FACS analysis**
   The wild type strain of *S. pombe* harbouring the plasmid expressing GFP gene under the control of *nmt1* promoter was grown upto mid-log phase at 37°C in presence of thiamine (promoter off) and then transferred to fresh medium lacking thiamine and further grown at 25°C. Samples were taken at 0, 3, 6, 9, 12, 15, 18 and 21hrs of induction and analyzed. 10,000 cells were analysed for fluorescence intensity using a Becton Dickson FACsort flow cytometer. Excitation was at 488nm and detection was through a 530-30nm filter. Counts: number of cells showing a given fluorescence. FL1-H: Fluorescence filter 1. For comparison, the background autofluorescence of the wild type strain is superimposed on the same profile.

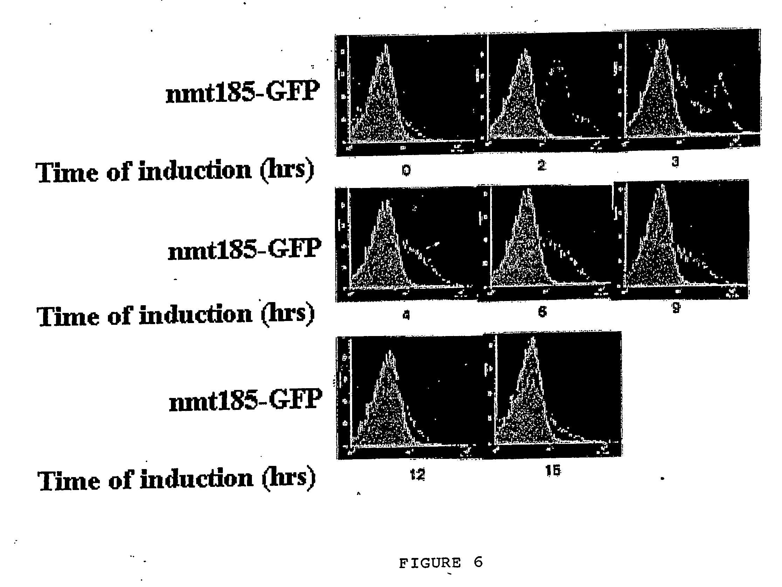

**Figure 6** **GFP expression under the control of *nmt-185* was monitored at cells level by FACS analysis**
   Wild type strain of *S. pombe* harbouring the plasmid expressing GFP gene under the control of *nmt-185* promoter was grown upto mid-log phase at 37°C (promoter off) and then transferred to fresh medium and further grown at 25°C to induce *gfp* exprssion. Samples were taken at 0, 2, 3, 4, 6, 9, 12 and 15 hours of induction and analyzed. 10,000 cells were analyzed for fluorescence intensity using a Becton Dickson FACsort flow cytometer. Excitation was at 488nm and detection was through a 530-30nm filter. Counts: number of cells showing a given fluorescence. FL1-H: Fluorescence filter 1. For comparison, the background autofluorescence of the wild type strain is superimposed on the same profile.

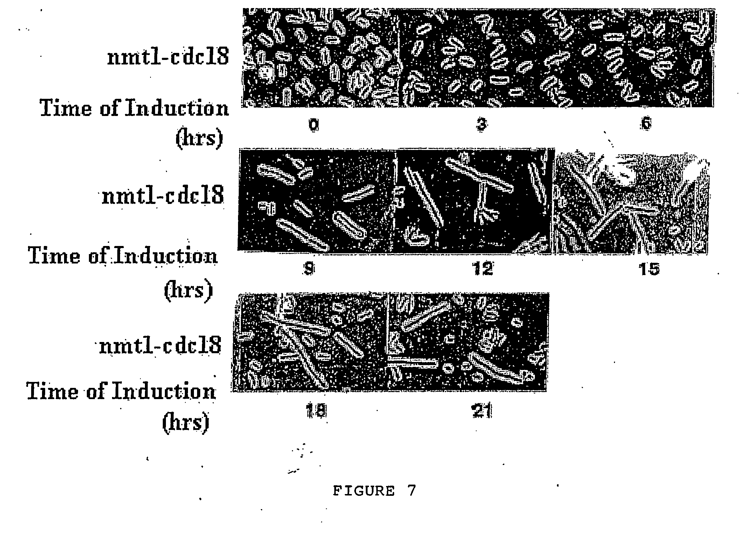

**Fig.7** **The effect of overexpression of *cdc*18 under the control of *nmt1* promoter**
   Overexpression of *cdc18* leads to elongation of wild type cells. The wild type strain of *S pombe* carrying *cdc18* under the control of *nmt1* were grown upto mid-log phase at 37°C in presence of thiamine (repressed) and then transferred to fresh medium lacking thiamine and further grown at 25°C (expressed). The samples were taken at times 0, 3, 6, 9, 12, 15, 18 and 21hrs of induction and seen under the phase contrast microscope.

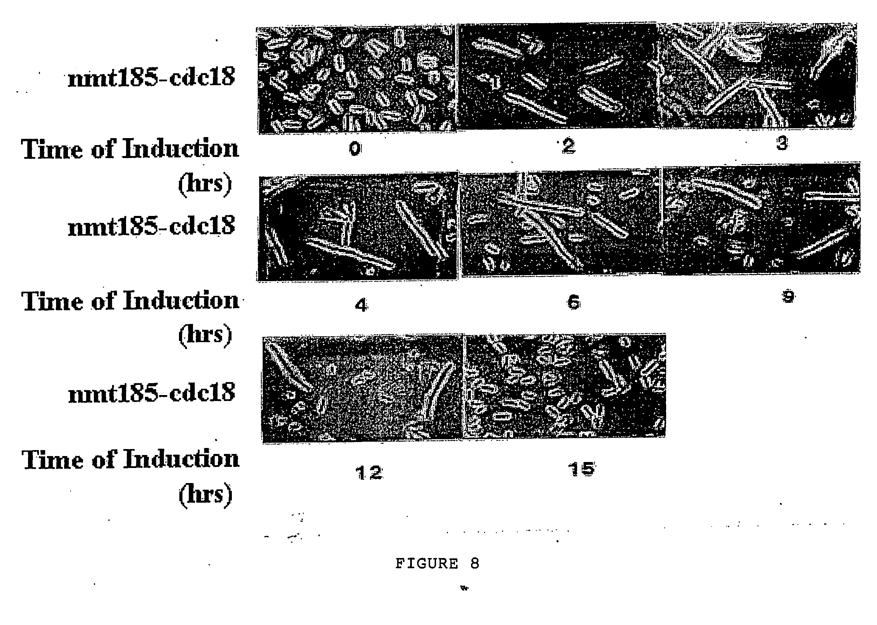

**Fig.8** **The effect of overexpression of *cdc18* under the control of *nmt-185* promoter**
   Wild type strain of *S. pombe* expressing *cdc18* under the control of *nmt*-*185* were grown upto mid-log phase at 37°C (repressed) and then transferred to fresh medium and grown further at 25°C (expressed). Samples were withdrawn after 0, 2, 3, 4, 6, 9, 12 and 15hrs of induction and observed under the phase contrast microscope.

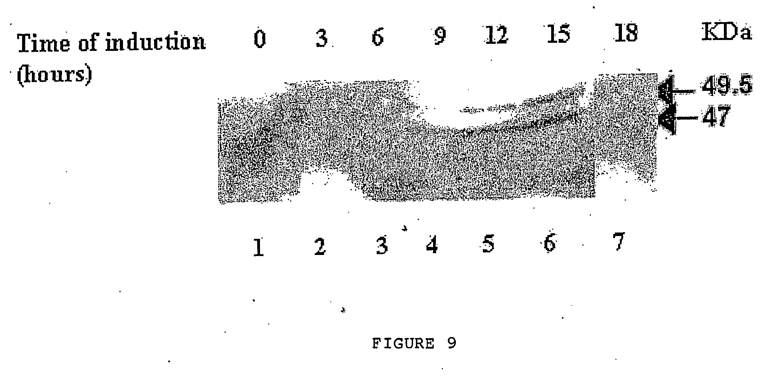

**Fig.9** **The Kinetics of SK expression under the control of *nmt*1 promoter**
   Whole cell extracts were prepared from the cells harboring recombinant plasmid pJRK1 (nmt1-SK; lanes 1-7) after 0, 3, 6, 9, 12, 15 and 18 hours of induction. The samples were subjected to SDS-PAGE (15%), Western blotted and probed with anti-SK polyclonal antibody. The unprocessed form (49.5kDa) and mature form of SK (47 kDa) are indicated by bar line.

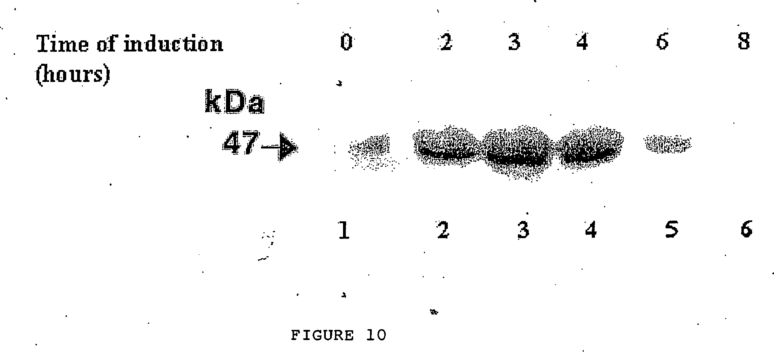

**Fig.10** **The Kinetics of SK expression under the control of *nmt*-185 promoter**
   Whole cell extracts were prepared from the cells containing vector pJRK2 (lanes 1-6) after induction at 25°C for different time points of induction. The samples were subjected to SDS-PAGE (12.5%), Western blotted and probed with anti-SK polyclonal antibody. Mature SK (47kDa) is indicated by arrows.

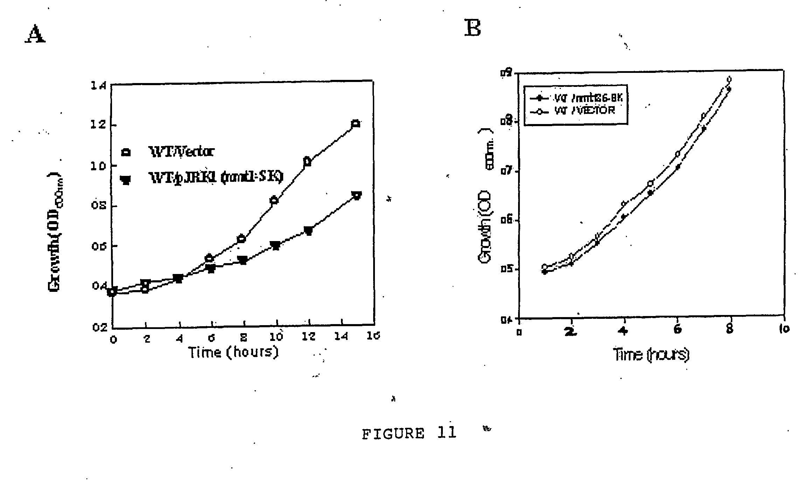

**Fig.11** **Effect of expression of SK under the control of nmt1 (A) and nmt185 (B) promoters on the growth rate of the S. pombe wild type strains**
   (A) Cells harboring vector (without promoter) or pJRK1 (*nmt*1-SK) were grown in PMA-leu medium containing thiamine for 18 hrs and further inoculated into fresh medium containing thiamine and grown upto OD₆₀₀ = 0.1. The culture was incubated for an additional 4-5 hrs at 30°C to reach the mid-log phase. Thiamine was removed by washing and the cells were grown further in the medium lacking thiamine at the same temperature. Samples were withdrawn at the indicated time points upto 15 hrs, their OD₆₀₀ recorded and plotted.
   (B) Cells harboring vector (without promoter) or *nmt*185-SK were grown in PMA-leu medium at 36°C for 18 hrs and further inoculated in fresh medium upto OD₆₀₀ =0.1. The culture was incubated for an additional 4-5 hrs at 36°C to reach the mid-log phase. Cells were then shifted to 25°C and grown further. Samples were withdrawn at the indicated time points upto 8 hrs, the OD₆₀₀ recorded and plotted
**Fig.12** **Fractionation of Sk expressed either with *nmt*1 or *nmt*-185 promoters:**
   (A & B) The periplasmic (PP) and cytoplasmic (CPP) fractions were prepared and subjected to SDS-PAGE (12.5%), Western blotted and probed with anti-SK polyclonal antibody lane 1, PP fraction, lane 2, CPP fraction; lane 3, pellet fraction of the wild type cells harboring plasmid pJRK1(*nmt*1-SK) or pJRK2-(*nmt*185-SK). The band of 45kD in the periplasmic fraction, which results from proteolysis, is indicated by arrow Note that the level is much higher in case of nmt1-SK (about 50%) than in case of nmt185-SK (∼5%).

### SUMMARY OF THE INVENTION

The process of the present invention involves construction of a genomic library of *S. pombe* upstream of the GFP-reporter gene in a suitable vector that contains the *ars*1 element and the *S. cerevisiae* β-isopropylmalate dehydogenase (*LEU2*) or the *S. pombe* orotidinephosphate decarboxylase *ura*4 gene as a selectable marker, and plating this library on appropriate media containing different metal salts or monovalent salts as inducers or growth at different ambient temperatures. The expression of GFP was monitored by exposure to UV light and the putative clones expressing GFP were subjected to more careful screening. From an extensive search, two promoter elements were isolated that allowed expression of the GFP reporter gene at 25°C but not at 36°C.

### DETAILED DESCRIPTION OF THE INVENTION

The advantages of the new promoters vis-a-vis the nmt1 and its other known derivative promoters strongly support the aspects of novelty and lack of obviousness and anticipation. In fact, literature published on the characteristics of the nmt1 promoters have only focussed on the minimal size of the nmt1 promoters that is repressible by thiamin and on the role of trans-acting factors. No investigation has envisaged or anticipated in published literature whether derivatives of the nmt1 promoter having altogether new characteristics different from the original parent promoter could be derived. In fact, from our screen we were expecting to isolate heat-inducible promoters, like heat shock promoters, rather than the temperature sensitive ones that we actually obtained. Thus, identification of promoter that is heat sensitive, was an unexpected discovery for us as well. Therefore, we believe that these points will strengthen the claims for lack of obviousness and anticipation in obtaining the present set of promoters.

For construction of the genomic library, genomic DNA was isolated from wild type *S. pombe* strain having no known auxotrophic markers. Partial digestion of the genomic DNA was carried out with *Sau3AI,* to obtain maximum amount of DNA in the range of 100 bp to 2000 bp. The *Sau3AI* digested genomic DNA was ligated to the unique *Bam*HI site in the vector pGFP (without *nmt*1 promoter) as per the standard procedure.

The above *S. pombe* 'promoter' library with GFP reporter was transformed into an *S. pombe* strain according to the published procedure of Gietz *et.al.,* 1992, *Nucleic acids Research* 20(6) : 1425. The yeast transformants were replica plated on PMA-leu plates containing different concentration of glucose, galactose, sodium chloride, copper and zinc. The same transformants were also replica plated on to PMA-leu plate in triplicate and incubated at different temperatures. Expression was followed under the different conditions by directly monitoring the green fluorescence of colonies when exposed to UV.

Two clones were isolated from the above screen that exhibited enhanced expression of GFP at 25°C and no expression at 36°C, as monitored by green fluorescence when exposed to the UV light.

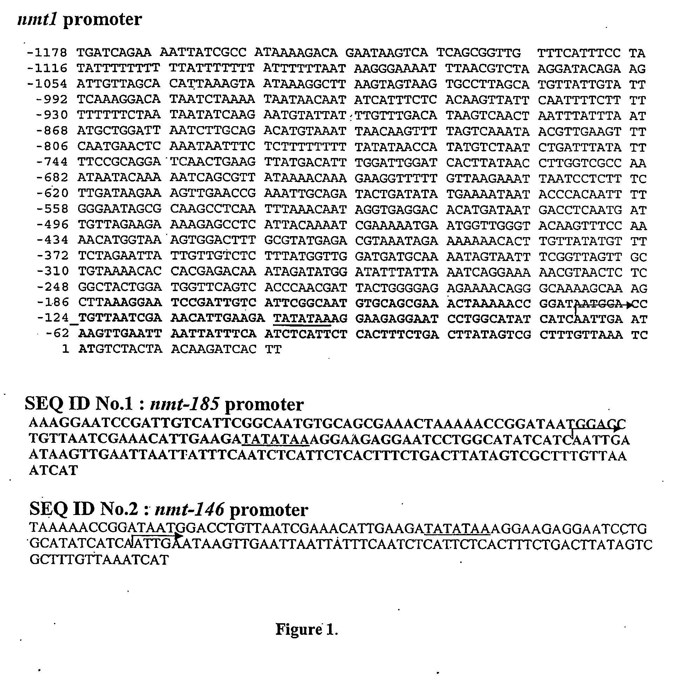

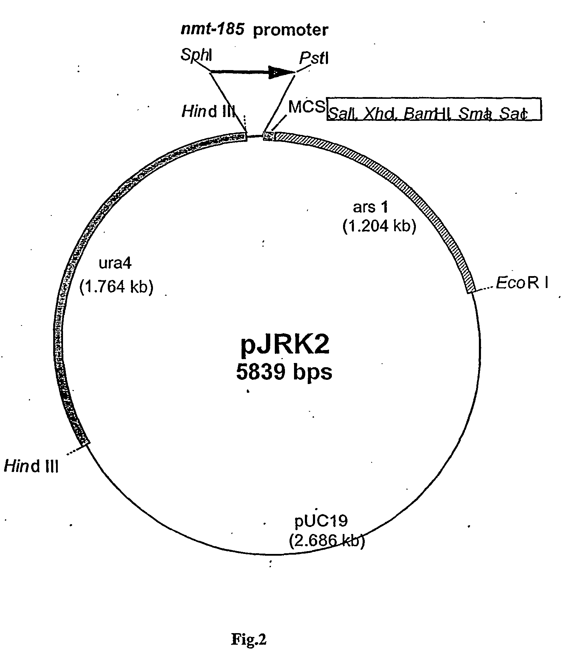

Automatic DNA sequencer (*PE* Applied Biosystems ABI 310) was used for sequencing of these clones. The DNA samples were processed for sequencing according to ABI Prism BigDye Terminator protocol (*PE* Applied Biosystems). The clones yielded sequences of 185 and 146 bases and, therefore, the new promoters were named as *nmt-185* (Fig. 1) and *mnt-146* (Fig. 2), respectively.

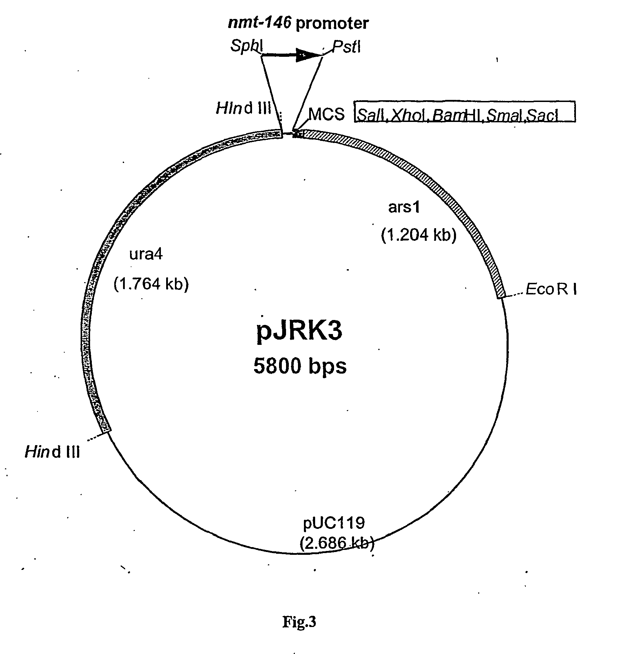

A new vector pJRK2 (Accession no. MTCC 5106) was designed in which the *nmt-185* promoter was cloned between the sites *Hind*III and *Pst*I, followed by multiple cloning sites (MCS) including *Sal*I, *Xho*I, *Bam*HI, *Sma*I and *Sac*I*,* into which other genes of interest could be cloned (Fig. 3).

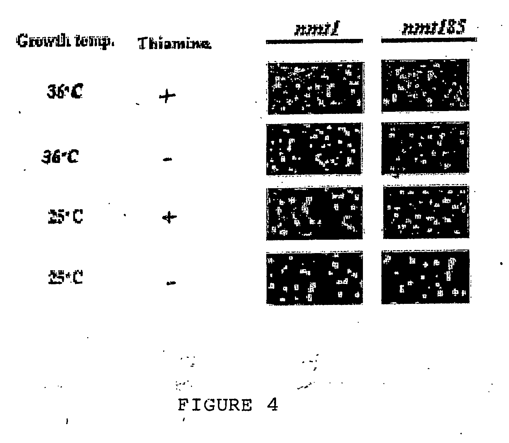

Another new vector pJRK3 (Accession no. MTCC 5107) was designed in which the *nmt-146* promoter was cloned between the sites *Hin*dIII and *Pst*I, followed by multiple cloning sites (MCS) including *Sal*I, *Xho*I, *Bam*HI, *Sma*I and *Sac*I, into which other genes of interest could be cloned (Fig. 4).

Because the new promoters show 100% homology with *mnt1* gene of *S. pombe,* experiments were carried out to check whether conditions of repression and expression by the new promoters are similar to or different from those with *nmt1* and to compare the strength as well as time of induction, using *gfp*-gene as a reporter gene. To compare the conditions of expression and repression of *nmt-185, nmt-146* and *mnt1* promoters, pGFP plasmids containing *nmt-185, nmt-146* or *nmt1* promoters were transformed into a wild type strain of *S*. *pombe.* GFP expression was monitored in the presence and absence of thiamine at 36°C and 25°C. It was observed that *mnt1, nmt-146* and *nmt-185* promoters were repressed in presence of thiamine at 36°C as monitored by green fluorescence. However, in absence of thiamine at 36°C, while *nmt1* promoter was derepressed, i.e., GFP gave green fluorescence, the *nmt-185* and *nmt-146* promoter remained repressed, i.e., GFP gene gave no green fluorescence. On the other hand at 25°C, *nmt1, nmt-185* and *nmt-146* were expressed as indicated by green fluorescence. When grown in absence of thiamine. But in presence of thiamine all three promoters were again repressed. Thus, the *nmt-185* and *nmt-146* promoters can be regulated by a temperature shift: they are repressed at 36°C, but expressed upon shift to 25°C in absence of thiamine. In contrast, the *nmt*1 promoter is equally expressed at 36°C and 25°C in absence of thiamine

A wild type strain of *S. pombe* carrying the clone for new promoters (*nmt-185* and *nmt-146*) upstream of GFP gene was grown in YEA medium at 33-37°C. Overnight cultures were reinnoculated into YEA media and grown further at 33-37°C to mid-log phase. They were washed thrice with sterile water, resuspended in PMA-leu media and grown further at 22-28°C for 1-5-hrs. Cells were collected by centrifugation, washed in sterile water, and fixed by suspending in water, after which it was diluted with ethanol and stored at low temperature indefinitely. The fixed cells were harvested for analysis by centrifugation, washed with water and resuspended in 50mM sodium acetate. Cells suspensions were sonicated briefly and analyzed using a FACSorting apparatus and data of fluorescence (FL1) plotted against forward scatter (FSC) or count against fluorescence (FL1).

Other genes, like β-galactosidase, Streptokinase from *S. equisimilis* and *cdc18* from *S. pombe* were cloned in front of the new promoter element *nmt-185* in the new vector pJRK2 (Accession no. MTCC 5106) (Fig. 3) and their expression was also monitored.

Other genes, like β-galactosidase, Streptokinase from *S. equisimilis* were also cloned in front of the new promoter element *nmt-146* in the new vector pJRK3 (Accession no. MTCC 5107) (Fig. 4) and their expression was also monitored.

Assay for β-galactosidase activity was carried out as described by Davenport et al., 1999 Genetics 153: 1091-1103.

Samples were withdrawn from cells expressing the *cdc*18 gene under the control of the *nmt-*185 promoter at different time points of induction and examined under the Phase-contrast microscope.

Wild type cells harboring the Streptokinase gene under the control of *nmt-185* or *nmt-146* promoters were grown in YEA medium upto mid-log phase and then inoculated into synthetic PMA medium lacking uracil to start the induction of SK expression. Samples were collected after different times of induction. To obtain cell extracts the harvested cells were washed once with chilled water and then with Buffer A (0.02M Hepes, pH

7.5, 0.1M Sodium chloride, 0.002M EDTA, 0.625% glycerol and 1mM β-mercaptoethanol). The cell pellet was resuspended in Buffer A containing protease inhibitors. An equal weight of acid-washed glass beads was added and cells broken by vortexing at 4°C. Each one min cycle of vortexing was followed by 30 sec incubation on ice till 80% lysis occurred. Cells lysate was centrifuged at 55,000 rpm for 1 hr at 4°C in TL-100-3 rotor of TL-100 ultracentrifuge. The supernatant (cell extract) was saved and stored at -70°C for further analysis.

Biologically active SK was quantitated as described by Jackson et al.,1981, Methods Enzymol. 80: 387-394.

Accordingly, the main embodiment of the present invention relates to novel temperature regulated promoters having SEQ ID No.1, designated as *nmt-185* and SEQ ID No.2, designated as *mnt-146.*

Another embodiment of the present invention relates to the novel temperature regulated expression vectors having Accession No. MTCC 5106 and MTCC 5107 deposited at International depository of Institute of Microbial Technology (IMTECH), Chandigarh, India, wherein,
(a) expression vector having Accession MTCC 5106 is harbouring temperature regulated promoter having SEQ ID No.1, designated as *nmt-185* and
(b) expression vector having Accession No. MTCC 5107 is harbouring temperature regulated promoter having SEQ ID No. 2, designated as *nmt-146*

Yet another embodiment of the present invention relates to the a process of isolating novel temperature regulated promoters from *Scizosaccharomyces pombe* said process comprising the steps of:
(a) constructing a partial genomic DNA library with restriction enzyme *Sau*3AI, to obtain partial genomic DNA sequences in the range of about 100 bp to 2000bp,
(b) ligating the genomic DNA library sequences of step (a) with vector pGFP without a promter
(c) transforming the vector of step (b) to *S.pombe* strain,
(d) screening of *S*. *pombe* strain containing the promoter library,
(e) isolating and identifying two clones of (step d) by stimulating GFP expression,
(f) using the clones obtained in step (e) to check repress or express of GFP expression by temperature shift,
(g) sequencing the genomic DNA fragments of (f) as new promoter elements having SEQ ID No. 1 and SEQ ID No.2, designating the promoters as *nmt-185* and *nmt-146,* useful as promoters,
(h) cloning the said promoter elements into the novel vectors having Accession nos. MTCC 5106 and 5107 respectively.

Still another embodiment of the present invention relates to a process of preparing novel expression vectors based temperature regulated novel promoter elements isolated from *Scizosaccharomyces pombe* said process comprising steps of:
(a) constructing a partial genomic DNA library with restriction enzyme *Sau*3AI, to obtain partial genomic DNA sequences in the range of about 100 bp to 2000bp,
(b) ligating the genomic DNA library sequences of step (a) with vector pGFP without a promter,
(c) transforming the vector of step (b) to *S. pombe* strain,
(d) screening of *S. pombe* strain containing the promoter library,
(e) isolating and identifying two clones of (step d) by stimulating GFP expression,
(f) using the clones obtained in step (e) to check repress or express of GFP expression by temperature shift,
(g) sequencing the genomic DNA fragments of (f) as new promoter elements having 185 bases and 146 bases, named as *nmt-185* and *mnt-146* respectively, useful as promoters, and
(h) cloning the said promoter elements into the novel vectors having Accession nos. MTCC 5106 and 5107 respectively.

One more embodiment of the present invention relates to a process wherein the step (f) the temperature shifts are 25°C and 37°C.

Yet another embodiment of the present invention relates to the promoters wherein said promoters have been isolated from *Schizosacchromyces pombe.*

Another embodiment of the present invention relates to promoter wherein the sequence of the said promoter element *nmt-185* and *nmt-146* is identical or more than 80% homologous to the sequence of *nmt1.*

Still another embodiment of the present invention relates to promoter wherein the promoter element *nmt-185* and *nmt-146* are repressed in the temperature range of about 33° to 37°C.

Yet another embodiment of the present invention relates to a promoter wherein the promoter element *nmt-185* and *nmt-147* are expressed in the temperature range of about 22° to 28°C.

One more embodiment of the present invention relates to a promoter wherein the promoter element *nmt- 185* is about 185 bases long.

Another embodiment of the present invention relates to a promoter wherein the promoter element *nmt- 146* is only 146 bases long.

Yet another embodiment of the present invention relates to the promoter wherein the promoter elements *nmt-186* and *nmt-145* can express or repress the genes GFP, Streptokinase, β-galactosidase and *cdc18 gene.*

Still another embodiment of the present invention relates to promoters wherein the GFP expression of said promoters is about 95 % within 3 hrs.

One more embodiment of the present invention relates to promoters wherein GFP expression of said promoters is about 91.4 % within 3 hrs.

Another embodiment of the present invention relates to promoters wherein said promoters have β-galactosidase activity of about 150 ± 20 units within 3 hrs of induction.

Still another embodiment of the present invention relates to promoters wherein said promoters have β-galactosidase activity of about 124.3 ± 20 units within 3 hrs of induction.

Yet another embodiment of the present invention relates to promoters wherein said promoters have maximum specific activity of about 900 I.U/mg in 3 hrs.

One more embodiment of the present invention relates to promoters wherein said promoters have maximum specific activity of about 870 ± 16 I.U/mg in 3 hrs.

Still another embodiment of the present invention relates to promoters wherein said promoters enhance expression of *cdc-18* gene within 3 hrs of induction.

Another embodiment of the present invention relates to promoters wherein said promoters give lower leaky expression of proteins.

One more embodiment of the present invention relates to promoters wherein said promoters are not deleterious to the cell viability.

Yet another embodiment of the present invention relates to promoters wherein said promoters reduce the level of proteolytic degradation.

The process of the present invention is illustrated in the examples given below which should not, however, be construed to limit the scope of the present invention.

### EXAMPLES

### Example 1: Isolation of Promoter elements

For search of promoters, a genomic library was constructed. For this, genomic DNA was isolated from wild type *S. pombe* strain having no known auxotrophic markers. Partial digestion of the genomic DNA was carried out with *Sau3AI,* to obtained maximum amount of DNA in the range of 100 bp to 2000 bp. The *Sau3AI* digested genomic DNA was ligated to the unique *Bam*HI site in the vector pGFP (without promoter).

The above *S. pombe* 'promoter' library with GFP reporter was transformed into *S. pombe* strain. The yeast transformants were replica plated on PMA-leu plates containing different concentration of glucose, galactose, sodium chloride, copper and zinc. The same transformants were also replica plated on to PMA-leu plate in triplicate and incubated at different temperatures. Expression was followed under the different conditions by directly monitoring the green fluorescence of colonies when exposed to UV.

Two clones were isolated from the above screen that exhibited enhanced expression of GFP at 25°C and no expression at 36°C, as monitored by green fluorescence when exposed to the UV light.

Automatic DNA sequencer (*PE* Applied Biosystems ABI 310) was used for sequencing of these clones. The DNA samples were processed for sequencing according to ABI Prism BigDye Terminator protocol (*PE* Appied Biosystems). The clones yielded sequences of 185 and 146 bases and, therefore, the new promoters were named as *nmt-185* (Fig. 1) and *nmt-146* (Fig. 1), respectively.

A new vector (Accession no. MTCC 5106) was designed in which the *nmt-185* promoter was cloned between the sites *Hind*III and *Pst*I, followed by multiple cloning sites (MCS) including *Sal*I*, -Xho*I, *Bam*HI*, Sma*I and *Sac*I, into which other genes of interest could be cloned (Fig. 2).

Another new vector (Accession no. MTCC 5107) was designed in which the *mnt-146* promoter was cloned between the sites *Hin*dIII and *Pst*I, followed by multiple cloning sites (MCS) including *Sal*I*, Xho*I, *Bam*HI, *Sma*I and *Sac*I*,* into which other genes of interest could be cloned (Fig. 3).

Both these vectors were deposited in International Depository Authority on Microbial Type Culture Collection and have given accession nos. MTCC 5106 and 5107 respectively.

### Example 2 : Expression of GFP by nmt-185 promoter

To monitor the expression level induced by the new promoter element, the gene encoding the green fluorescent protein (GFP) was cloned in the MCS of the vector pJRK2 (Accession no. MTCC 5106) (Fig.3). The same gene was also cloned in front of the *nmt1* promoter for comparison of level of expression. Flow cytometry analysis was used to quantify the amount of GFP protein expressed under the control of *mnt-185* at different time intervals of induction. As yeast cells have some background autofluorescence when irradiated at 488 nm, the fluorescence profile of *S. pombe* strain having control vector was analyzed first. *S. pombe* cells were grown to mid-exponential phase in rich medium under repressed conditions (36°C) and then shifted to expressed condition in a selective medium (25°C). Samples were taken at 0, 2, 3, 4, 6, 9, 12, and 15 hours of induction and the fluorescence profile of 10,000 cells analyzed. In case of the *mnt-185* promoter, the fluorescence signal was maximum after a 3 hr period of induction, followed by a decrease with further increase in time of induction.

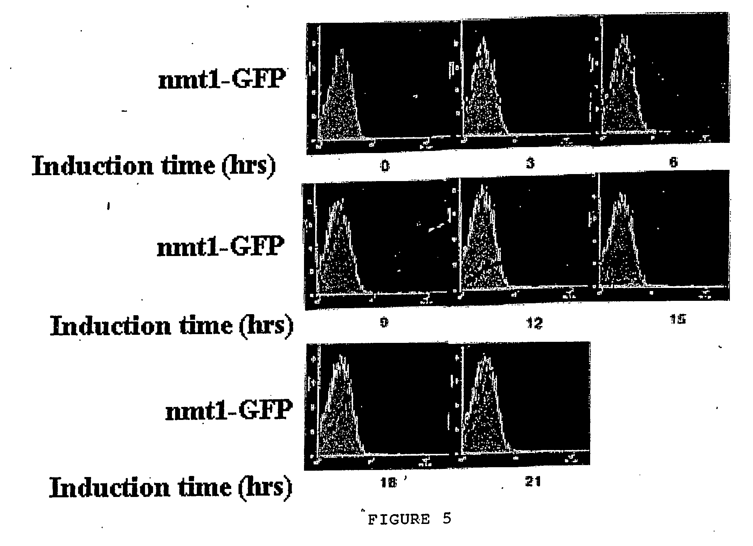

The study of the present shows that nmt-185 promoter also gives maximum GFP expression after 3 hours of induction, while nmt1 promoter does so only after 15 hours of induction. Furthermore, the level of expression appears to be similar in both cases as indicated by similar fraction of cells expressing GFP by both the promoters (**Figures 4 to 6****; Table 1**).

**Table 1 Levels of GFP expression under the control of nmt-185 and nmt1 promoters. Samples drawn at different time points of induction were analyzed by FACS.***

| **Sr. No.** | **Time (Hrs)** | **GFP expression level (*nmt-185* promoter)** | | **GFP expression level (*nmt1* promoter)** | |
|---|---|---|---|---|---|
| | | **% non-expressing cells** | **%Expressing cells** | **%non-expressing cells** | **%expressing cells** |
| 1 | Control | 98.55 | 1.45 | 99.01 | 1.01 |
| 2 | 0 | 97.84 | 2.16 | 98.98 | 1.04 |
| 3 | 2 | 63.03 | 36.97 | - | - |
| 4 | 3 | 8.60 | 91.4 | 98.26 | 1.74 |
| 5 | 4 | 72.57 | 27.43 | - | - |
| 6 | 6 | 71.86 | 28.14 | 94.42 | 5.86 |
| 7 | 9 | 77.81 | 22.19 | 89.54 | 10.46 |
| 8 | 12 | 89.40 | 10.6.0 | 29.74 | 71.26 |
| 9 | 15 | 95.12 | 4.88 | 4.72 | 95.28 |
| 10 | 18 | - | - | 53.21 | 47.34 |
| 11 | 21 | - | - | 58.83 | 42.17 |

| | | | | | |
|---|---|---|---|---|---|
| * This was calculated by using Kolmogarov-Smirnov Statistics Programme. - denotes for not determined. | | | | | |

### Example 3: Expression of cdc18 gene of S. pombe by nmt-185 promoter

The new promoter element *nmt*-185 isolated from the promoter screen was also used to monitor the expression of cell division cycle gene *cdc18* from *S. pombe.* Overproduction of *cdc*18 has been shown to cause delay in mitosis and more than one round of DNA synthesis leading to a drastic elongation of cells. In the present example expression of *cdc18*⁺ gene was carried out under the control of *nmt-185* promoter.

The *cdc18* gene was cloned into the multiple cloning sequence (MCS) of the vector pJRK2 (Accession no. MTCC 5106) and transformed in to a wild type strain of S. *pombe.* Similarly, the *cdc18* gene was also cloned in front of the *nmt1* promoter. Cells were grown to exponential phase in medium containing thiamine (promoter off) at 36°C, washed extensively and transferred to fresh media without thiamine at 25°C (promoter on) to induce the expression of *cdc18.* Samples were withdrawn after 0, 2, 3, 4, 6, 9, 12, and 15 hours of induction and examined under the Phase-contrast microscope. In case of *nmt-185* promoter, cells start becoming elongated after 2 hrs of induction and achieved maximum elongation after 3 hrs. Further, see the data for expression of cdc18 by nmt1 and nmt-185 promoters in and Figs. 7 and 8, respectively. These data also indicate that maximum effect of overexpression of cdc18, visualized as fraction of elongated cells, is achieved by 3 hours with nmt-185 promoter (Fig. 7), while it takes 15-18 hours when expressed under the control of nmt1 promoter (Fig. 7)

### Example 4: Expression of β-galactosidase by the nmt-185 promoter

The *Pst*I*-Xho*I fragment containing the *nmt*-185 promoter was cloned into the *Pst*I and *Xho*I sites of the vector pREP3X- *lacZ* in place of the *nmt1* promoter (Maundrell, 1990, J. Biol. Chem. 265: 10857-10864). Thus, the *lacZ* gene was expressed under control of *nmt-185* promoter and the β-galactosidase activity monitored at 0, 2, 3, 4, 6, 9, 12 and 15 hours of induction using ONPG as substrate. Activity was maximum after 3hrs of induction in case of *nmt-185* promoter. The *nmt-185* promoter induced β-galactosidase by approximately 25-fold. See comparison of expression of (5-galactosidase by pREPS, pREP41, pREP81 and pREP-185 in Tables 2 and 3. The data show that pREP-185 promoter allows expression at the same level as that shown by the promoter pREP41, i.e., moderate level of expression. Furthermore, it shows a similarly low level of leaky expression as pREP41 under repressed condition. However, the nmt-185 promoter is better than even nmt1, which is the strongest version of nmt promoter, in that it shows maximum expression after only 3 hours of induction (Table 3), while nmt1 gives maximum expression only after 15 hours of induction.

**Table 2 Comparison of β-galactosidase activity of known promoters of S. pombe with nmt-185 promoter**

| **Vector** | **Promoter** | **Repressed conditions In Units** | **Induced conditions In Units** | **Approx. Induction Ratio** | **Ref.** |
|---|---|---|---|---|---|
| pREP3X-*lacZ* | *mnt1* (full strength) | 24.7±9 | 7395±404 | 300X | |
| pREP41X-*lacZ* | *nmt1* (weaker) | 5.1±1 | 121±3 | 25X | Forsburg, 1993. |
| pREP81X-*lacZ* | *mnt1* (weakest) | 1.2±0.3 | 7.2±2 | 7X | |
| pREP3X-*lacZ* | *nmt1* (full strength) | 7.59±1.3 | 2053±65 | 271X | **Present Invention** |
| pREP3/185-*lacZ** | *nmt-185* | 5.4±1.0 | 124.3±20 | 25X | **Present Invention** |

| | | | | | |
|---|---|---|---|---|---|
| ** nmt1* promoter fragment of vector pREP3X-*lacZ* was replaced with the *nmt-185* promoter fragment. | | | | | |

**Table 3 Levels of β-galactosidase activity expressed at different times of induction of lacZ gene when expressed under the control of nmt1 and nmt-185 promoters.**

| **Sr. No.** | **Induction time** | **β-gal activity** | **β-gal activity** |
|---|---|---|---|
| | (Hrs) | (*nmt-185* promoter) (Units) | *(nmt1* promoter) (Units) |
| 1. | 0 | 5.4±1.0 | 7.59±1.3 |
| 2. | 2 | 56.8±12 | - |
| 3. | 3 | 124.3±20 | 8.65±1.2 |
| 4. | 4 | 29.0±12 | - |
| 5. | 6 | 16.25±6.2 | 42.65±11.8 |
| 6. | 9 | 13.82±5.2 | 130.02±12.6 |
| 7. | 12 | 13.67±6.4 | 1845.2±45 |
| 8. | 15 | 10.23±3.4 | 2053±65 |
| 9. | 18 | - | 1025±42 |
| 10. | 21 | - | 825±20 |

| | | | |
|---|---|---|---|
| - denoted for not determined. | | | |

### Example 5: Expression of Streptokinase by the nmt-185 promoter

To check whether a heterologous gene can be expressed by the new promoter, a chimeric gene construct comprising a fusion between the Plus pheromone signal sequence of *S*. *pombe* and the mature SK was cloned in front of the *nmt-185* promoter in the vector pJRK2 (Accession no. MTCC 5106). The cloned plasmid was transformed into a wild type strain of *S*. *pombe* and the transformants were selected for uracil prototrophy at 37°C. To assess the level of SK activity, plasminogen clearing plate assay was performed by replica plating, overlaying with skim milk-agarose containing plasminogen and incubating at 25°C. After 4 hrs, the transformants harboring the plasmid expressing SK gene produced a clear zone. Thus, the recombinant plasmid harboring the Streptokinase gene under the control of *nmt-185* promoter also expresses SK activity in *S. pombe.*

To obtain the optimum conditions for expression using the new promoter, wild type cells expressing the recombinant plasmid pJRK2 (accession no. MTCC 5106) containing the chimeric Plus- factor-SK fusion gene were grown in YEA medium at 37°C upto mid-log phase and then inoculated into synthetic PMA medium lacking uracil at 25°C to start the induction of SK expression. Samples were collected after 2, 3, 4, 6 and 8 hrs of induction. At the same time points culture supernatants were also collected and concentrated. No SK activity was observed in supernatant. Cell extracts were subjected to SDS-polyacrylamide gel electrophoresis, followed by immunoblotting with anti-SK antibody. One band of 47kDa corresponding to mature SK could be observed. SK activity in the extract was quantitated using chromozyme substrate and maximum level of specific activity of 870 I.U./mg protein was observed after 3 hrs of induction. The activity decreased significantly after 4 hrs of induction and no activity could be detected after 6 and 8 hrs of induction. No activity could be detected in the culture supernatant at any time point. The data for expression of streptokinase by nmt1 in **Fig. 9** and that by nmt-185 promoter in **Fig. 10** and the comparison of the maximum activities using the two promoters and the time of induction in **Table 4.** These data again show that nmt-185 promoter yields maximum expression of SK within 3 hours of induction, as compared with 15 hours required by the nmt1 promoter **(Table 5).**

Although nmtl remains expressed over a wide temperature range, its benefit is counterbalanced because of the long induction period, toxicity, metabolic load and cumbersome handling required to remove the represser (thiamin) by repeated washings. On the other hand in case of the new promoters, the induction step involving reducing the temperature from 36°C to 25°C is operationally easy to perform, without a change of medium. Along with other advantages, like reduced induction period (from 15hrs with nmt1 to 3 hours with nmt185/nmt146), no effect on growth rate and reduced level of toxicity and proteolysis, possibly because of the reduced time of exposure of the expressed protein, makes the new promoters much more advantageous to use (This has been discussed as below).

The inventors have performed experiments to compare the growth rates of cultures expressing Streptokinase (SK) under the control of nmt1 and nmt185 promoters and find that while in the case of nmt1 -induced expression there is 50% reduction in growth rate accompanying the induction of SK expression (compare **Figure 11** with **Figure 9**), there is no effect on growth when SK was expressed under the control of nmt185 (Compare **Figure 11** with **Figure 10**). The **Figure 11** is suggesting lack of toxicity and/or reduction of growth rate while using nmt-185 promoter in contrast with nmt1 promoter.

Likewise, while significant level of proteolysis of SK is observed in the periplasmic fraction of cells expressing SK under the control of nmt1 promoter, less than 5% proteolysis of SK was observed in the periplasmic fraction of cells expressing SK under the control of nmt185 promoter. Thus, expression with the nmt-185 promoter results in very much reduced proteolysis in the periplasmic fraction as compared to expression by the nmt1 promoter, which may be due to reduced time of exposure of protein .to the extracellular or periplasmic proteases. This data is shown in Figure **12****.**

**Table 4: Comparison of maximum Specific activity of SK achieved when expressed under the control of nmtl and nmt185 promoters**

| Time **(Hrs)** | Specific activity (I.U./mg) | |
|---|---|---|
| | ***nmt1*** | ***nmt* 185** |
| **3** | **-** | **870±16** |
| **15** | **1581±80** | - |

| | | |
|---|---|---|
| - denotes no detectable SK activity. | | |

**Table 5 Comparison of Expression levels of different genes under the control of nmt1 and nmt-185 promoter.**

| **Sr. No.** | **Gene Expressed** | ***nmt-185* promoter** | | ***nmt1* promoter** | |
|---|---|---|---|---|---|
| | | **Time of Induction** (hrs) | **Level of expression** (max.) | **Time of Induction** (hrs) | **Level of expression** (max.) |
| 1. | *gfp* | 3 | 91.4%^{@} | 15 | 95.28%^{@} |
| 2. | *SK* | 3 | 870IU/mg | 15 | 1581 IU/mg |
| 3. | β*-gal* | 3 | 124 U | 15 | 2053 U |
| *4.* | *cdc18* | 3 | max. elongated cells | 15 | max. elongated cells |

| | | | | | |
|---|---|---|---|---|---|
| ^{@} - % age of cells expressing gfp. U - units I.U. - International units | | | | | |

### Example 6: Expression of β-galactosidase by the nmt-146 promoter

The *Pst*I*-Xho*I fragment containing the *nmt*-146 promoter was cloned into the *Pst*I and *Xho*I sites of the vector (pREP3X-*lac*Z) in place of the *nmt1* promoter (Maundrell, 1990, J. Biol. Chem. 265: 10857-10864). Thus, the *lacZ gene* was expressed under control of *nmt-146* promoter and the β-galactosidase activity monitored at 0, 2, 3, 4, 6, 9, 12 and 15 hours of induction using ONPG as substrate. Activity was maximum after 3hrs of induction in case of *nmt-146* promoter. Like *nmt-185,* the *nmt-146* promoter also induced β-galactosidase by approximately 25-fold.

### Example 7: Expression of Streptolcinase by the nmt-146 promoter

To check whether a heterologous gene can be expressed by the new promoter, a chimeric gene construct comprising a fusion between the Plus pheromone signal sequence of *S. pombe* and the mature SK was cloned in front of the *nmt-146* promoter in the vector pJRK3 (Accession no. MTCC 5107)(Fig. 4). The cloned plasmid was transformed into a wild type strain of *S*. *pombe* and the transformants were selected for uracil prototrophy at 37°C. To assess the level of SK activity, plasminogen clearing plate assay was performed by replica plating, overlaying with skim milk-agarose containing plasminogen and incubating at 25°C. After 4 hrs, the transformants harboring the plasmid expressing SK gene produced a clear zone. Thus, the recombinant plasmid harboring the Streptokinase gene under the control of *nmt-146* promoter also expresses SK activity in *S. pombe* at levels similar to those obtained with the *nmt-185* promoter.

### THE ADVANTAGES OF THE NEW PROMOTERS NMT-185 AND NMT1-46

➢ *Faster induction kinetics of the nmt-185 and nmt-146 promoters as compared to the nmt1 promoter:* The *nmt*1 promoter provides maximum expression level after 18-20 hours of induction, while the *mnt-185* and *nmt-146* do so within 3. hours of induction- thus providing a 5-6 fold faster rate of induction than the *nmt*1 promoter.
➢ *this minimizes the problem of metabolic load.* The faster kinetics of induction in case of the *mint-185* and *mnt-146* promoters has another advantage that maximum level of expression of protein is achieved within less than one generation (5 hours), obivating the posulated of exerting a metabolic load on the host cells.
➢ *Low level of leaky expression of the protein:* We fmd that the *nmt-185* and *mnt-146* promoters give much lower leaky expression levels as compared to the *nmt*1 promoter but similar to that observed with the *nmt*41, a moderately active derivative of the *nmt*1 promoter. This aspect provides for tighter control of expression.
➢ *Ease and reduced cost of manipulation:* Operationally, the temperature shift from 36°C (repressed) to 25°C (expressed) is easy to perform and convenient for bench scale experiments and commercial process, as it significantly reduces the cost and time factor. In contrast, manipulations with the *nmt*1 promoter involve extensive washings to remove the repressor (thiamine) and suspending the cells in thiamin-free medium; these operations add to the cost of expression and production.
➢ *Minimum deleterious effect on cell viability* The short time of induction, which is less than one generation time of the growing cells, minimizes the cells' exposure to the toxic effects of the protein.
➢ *Reduced level of proteolytic degradation.* The reduced time of exposure of the expressed protein to the cellular environment before being harvested also results in reduced level of degradation by the intracellular proteases.

### SEQUENCE LISTING

### General Information

Applicant COUNCIL OF SCIENTIFIC & INDUSTRIAL RESEARCH
Title of Invention : NOVEL TEMPERATURE REGULATED PROMOTERS AND EXPRESSION VECTORS
Number of Sequences : 2
Correspondence Address : Institute of Microbial Technology, India Sector -39A, Chandigarh-160036, India
**INFORMATION FOR SEQ ID No:**1
**SEQUENCE CHARACTERISTICS:** *nmt-185* promoter
   **LENGTH:** 185 bases
**TYPE:** DNA 
5'AAAGGAATCCGATTGTCATTCGGCAATGTGCAGCGAAACTAAAAACCGGAT
AATGGACCTGTTAATCGAAACATTGAAGATATATAAAGGAAGAGGAATCCTG
GCATATCATCAATTGAATAAGTTGAATTAATTATTTCAATCTCATTCTCACTTT
CTGACTTATAGTCGCTTTGTTAAATCAT3'
**ORGANISM:** *Schizosacchromyces pmbe*
   **IMMIDIATE:** Natural sequence
   **NAME/KEY:** N.A
   **SEQUENCE ID** # 1
**INFORMATION FOR SEQ ID No:** 2
**SEQUENCE CHARACTERISTICS:** *nmt*-*146*promoter
   **LENGTH:** 146 bases
**TYPE:** DNA 
5'TAAAAACCGGATAATGGACCTGTTAATCGAAACATTGAAGATATATAAGGAAGAG
GAATCCTGGCATATCATCAATTGAATAAGTTGAATTAATTATTTCAATCTCATTCTCAC
TTTCTGACTTATAGTCGCTTTGTTAAATCAT3'
**ORGANISM:** *Schizosacchromyces pmbe*
   **IMMEDIATE:** Natural sequence
   **NAME/KEY:** N.A
   **SEQUENCE ID** # 2

## Claims

1. Novel temperature regulated promoters having SEQ ID No. 1 as depicted in figure 1, designated as *nmt-185* or SEQ ID No. 2 as depicted in figure 1, designated as *nmt-146.*

2. Novel temperature regulated expression vectors having Accession No. MTCC 5106 and MTCC 5107 deposited at International depository of Institute of Microbial Technology (IMTECH), Chandigarh, India, wherein,
(a) expression vector having Accession MTCC 5106 is harbouring temperature regulated promoter having SEQ ID No. 1 as depicted in figure 1, designated as *nmt-185* and
(b) expression vector having Accession No. MTCC 5107 is harbouring temperature regulated promoter having SEQ ID No. 2 as depicted in figure 1, designated as *nmt-146.*

3. A process of isolating novel temperature regulated promoters from *Schizosaccharomyces pombe* said process comprising the steps of:
(a) constructing a partial genomic DNA library Schizosaccharomyces pombe with restriction enzyme *Sau*3AI, to obtain partial genomic DNA sequences in the range of about 100 bp to 2000 bp,
(b) ligating the genomic DNA library sequences of step (a) with vector pGFP without a promoter
(c) transforming the vector of step (b) to *S. pombe* strain,
(d) screening of *S. pombe* strain containing the promoter library for the transformants that give GFP expression upon a temperature shift,
(e) isolating and identifying two clones of (step d) identified after stimulating GFP expression upon subjecting the transformant colonies to temperature shift,
(f) sequencing the genomic DNA fragments of (f) as new promoter elements having SEQ ID No. 1 or SEQ ID No. 2 as depicted in figure 1, designating the promoters as *nmt-185* and *nmt-146,* useful as promoters, and
(g) cloning the said promoter elements into the novel vectors having Accession nos. MTCC 5106 and 5107 respectively.

4. A process of preparing novel expression vectors based temperature regulated novel promoter elements isolated from *Schizosaccharomyces pombe* said process comprising steps of:
(a) constructing a partial genomic DNA library Schizosaccharomyces pombe with restriction enzyme *Sau*3AI, to obtain partial genomic DNA sequences in the range of about 100 bp to 2000 bp,
(b) ligating the genomic DNA library sequences of step (a) with vector pGFP without a promoter
(c) transforming the vector of step (b) to *S. pombe* strain,
(d) screening of *S. pombe* strain containing the promoter library for the transformants that give GFP expression upon temperature shift,
(e) isolating and identifying two clones of (step d) identified after by stimulating GFP expression upon subjecting the transformant colonies to temperature shift,
(f) sequencing the genomic DNA fragments of (f) as new promoter elements of 185 bases having SEQ ID No. 1 or 146 bases having SEQ ID No. 2 as depicted in figure 1, designated as *nmt-185* and *nmt-146* respectively, and
(g) cloning the said promoter elements into the novel vectors having Accession vector nos. MTCC 5106 and 5107 respectively.

5. A process as claimed in claim 4 or claim 5, wherein the step (f) the temperature shifts are 25°C and 37°C.

## Patentansprüche

1. Neue temperaturgeregelte Promotoren mit der SEQ ID Nr. 1, wie in Fig. 1 dargestellt, mit der Bezeichnung *nmt-185,* oder SEQ ID Nr. 2, wie in Fig. 1 dargestellt, mit der Bezeichnung nmt-*146.*

2. Neue temperaturgeregelte Expressionsvektoren mit der Hinterlegungsnummer MTCC 5106 und MTCC 5107, die beim International Depository des Institute of Microbial Technology (IMTECH), Chandigarh, Indien, hinterlegt sind, wobei
(a) der Expressionsvektor mit der Hinterlegungsnummer MTCC 5106 einen temperaturgeregelten Promotor mit der SEQ ID Nr. 1, wie in Fig. 1 dargestellt, mit der Bezeichnung *nmt*-*185* enthält, und
(b) der Expressionsvektor mit der Hinterlegungsnummer MTCC 5107 einen temperaturgeregelten Promotor mit der SEQ ID Nr. 2, wie in Fig. 1 dargestellt, mit der Bezeichnung *nmt-146* enthält.

3. Verfahren zur Isolierung neuer temperaturgeregelter Promotoren aus *Schizosaccharomyces pombe*, welches Verfahren die Schritte umfasst:
(a) Konstruieren einer teilweisen genomischen DNA-Bibliothek von *Schizosaccharomyces pombe* mit dem Restriktionsenzym Sau3AI zum Erhalt von teilweisen genomischen DNA-Sequenzen im Bereich von etwa 100 bp bis 2000 bp,
(b) Ligieren der genomischen DNA-Bibliothek-Sequenzen aus Schritt (a) mit dem Vektor pGFP ohne Promotor,
(c) Transformieren des Vektors aus Schritt (b) in den *S. pombe*-Stamm,
(d) Screenen des *S. pombe*-Stamms, der die Promotor-Bibliothek enthält, auf Transformanten, die nach einer Temperaturverschiebung eine GFP-Expression liefern,
(e) Isolieren und Identifizieren zweier Klone aus Schritt (d), welche nach Stimulieren der GFP-Expression, nachdem die Transformanten-Kolonien einer Temperaturverschiebung unterzogen worden waren, identifiziert wurden,
(f) Sequenzieren der genomischen DNA-Fragmente aus (e) als neue Promotor-Elemente mit der SEQ ID Nr. 1 oder SEQ ID Nr. 2, wie in Fig. 1 dargestellt, wobei die Promotoren als *nmt-185* und *nmt-146* bezeichnet werden, die als Promotoren geeignet sind, und
(g) Klonieren der Promotorelemente in die neuen Vektoren mit der Hinterlegungsnummer MTCC5106 bzw. 5107.

4. Verfahren zur Herstellung neuer Expressionsvektoren auf Basis von temperaturregulierten, neuen, aus *Schizosaccharomyces pombe* isolierten Promotor-Elementen, welches Verfahren die Schritte umfasst:
(a) Konstruieren einer teilweisen genomischen DNA-Bibliothek von *Schizosaccharomyces pombe* mit dem Restriktionsenzym *Sau*3AI zum Erhalt von teilweisen genomischen DNA-Sequenzen im Bereich von etwa 100 bp bis 2000 bp,
(b) Ligieren der genomischen DNA-Bibliothek-Sequenzen aus Schritt (a) mit dem Vektor pGFP ohne Promotor,
(c) Transformieren des Vektors aus Schritt (b) in den *S. pombe*-Stamm,
(d) Screenen des *S. pombe*-Stamms*,* der die Promotor-Bibliothek enthält, auf Transformanten, die nach einer Temperaturverschiebung eine GFP-Expression liefern,
(e) Isolieren und Identifizieren zweier Klone aus Schritt (d), welche nach Stimulieren der GFP-Expression, nachdem die Transformanten-Kolonien einer Temperaturverschiebung unterzogen worden waren, identifiziert wurden,
(f) Sequenzieren der genomischen DNA-Fragmente aus (e) als neue Promotor-Elemente von 185 Basen mit der SEQ ID Nr. 1, wie in Fig. 1 dargestellt, oder 146 Basen mit der SEQ ID Nr. 2, wie in Fig. 1 dargestellt, wobei die Promotoren als *nmt-185* und *nmt-146* bezeichnet werden, und
(g) Klonieren der Promotorelemente in die neuen Vektoren mit der Hinterlegungsnummer MTCC5106 bzw. 5107.

5. Verfahren nach Anspruch 4 oder Anspruch 5, wobei in schritt (f) die Temperaturverschiebungen 25°C und 37°C sind.

## Revendications

1. Promoteurs régulés selon la température d'un nouveau type ayant SEQ ID No. 1 tel que représenté sur la figure 1, désigné nmt-185 ou SEQ ID No. 2 tel que représenté sur la figure 1 désigné nmt-146.

2. Vecteurs d'expression régulés selon la température d'un nouveau type ayant les No. Accession MTCC 5106 et MTCC 5107 déposés à l'International depository de l'Institute of Microbial Technology (IMTECH), Chandigarh, Inde, dans lesquels,
(a) une vecteur d'expression ayant le No d'Accession MTCC 5106 héberge le promoteur régulé selon la température ayant la SEQ ID No. 1 tel que représenté sur la figure 1, désigné nmt-185 et
(b) un vecteur d'expression ayant le No d'Accession MTCC 5107 héberge le promoteur régulé selon la température ayant la SEQ ID No. 2 tel que représenté sur la figure 1, désigné nmt-146.

3. Procédé d'isolation de promoteurs régulés selon la température d'un nouveau type à partir de Schizosaccharomyces pombe, ledit procédé comprenant les étapes consistant à :
(a) construire une banque d'ADN génomique partielle Schizosaccharomyces pombe avec l'enzyme de restriction Sau3AI, pour obtenir les séquences d'ADN génomique partielles dans l'intervalle d'environ 100 bp à 2000 bp,
(b) ligaturer les séquences de la banque d'ADN génomique de l'étape (a) avec le vecteur pGFP sans promoteur
(c) transformer le vecteur de l'étape (b) en souche s. pombe,
(d) cribler la souche S. pombe contenant la banque de promoteur pour les transformants qui donnent une expression de GFP lors d'un décalage de température,
(e) isoler et identifier deux clones de (étape d) identifiés après stimulation de l'expression de GFP en soumettant les colonies de transformants à un décalage de température,
(f) séquencer les fragments d'ADN génomique de (f) sous forme de nouveaux éléments promoteurs ayant SEQ ID No. 1 ou SEQ ID No.2 tel que représenté sur la figure 1, désignant les promoteurs nmt-185 et nmt-146, utiles en tant que promoteurs, et
(g) cloner lesdits éléments de promoteur en les vecteurs d'un nouveau type ayant les No. Accession MTCC 5106 et 5107 respectivement.

4. Procédé de préparation d'éléments de promoteur d'un nouveau type régulés selon la température à base de vecteurs d'expression d'un nouveau type isolés à partir de Schizosaccharomyces pombe, ledit procédé comprenant les étapes consistant à :
(a) construire une banque d'ADN génomique partielle Schizosaccharomyces pombe avec l'enzyme de restriction Sau3AI, pour obtenir les séquences d'ADN génomique partielles dans l'intervalle d'environ 100 bp à 2000 bp,
(b) ligaturer les séquences de la banque d'ADN génomique de l'étape (a) avec le vecteur pGFP sans promoteur
(c) transformer le vecteur de l'étape (b) en souche S. pombe,
(d) cribler la souche S. pombe contenant la banque de promoteur pour les transformants qui donnent une expression de GFP lors d'un décalage de température,
(e) isoler et identifier deux clones de (étape d) identifiés après stimulation de l'expression de GFP en soumettant les colonies de transformant à un décalage de température,
(f) séquencer les fragments d'ADN génomique de (f) sous forme de nouveaux éléments promoteurs ayant SEQ ID No. 1 tel que représenté sur la figure 1 ou 146 bases ayant SEQ ID No. 2 tel que représenté sur la figure 1, désignés nmt-185 et nmt-146 respectivement, et
(g) cloner lesdits éléments de promoteur en les vecteurs d'un nouveau type ayant les No. Accession de vecteurs MTCC 5106 et 5107 respectivement.

5. Procédé selon la revendication 4 ou la revendication 5, dans lequel dans l'étape (f) les décalages de température sont 25°C et 37°C.
